(19) Europäisches Patentamt
European Patent Office
Office européen des brevets

(11) **EP 4 800 165 A1**

(12) **EUROPEAN PATENT APPLICATION**
published in accordance with Art. 153(4) EPC

(43) Date of publication:
**02.09.2026 Bulletin 2026/36**

(21) Application number: **24882322.1**

(22) Date of filing: **21.10.2024**

(51) International Patent Classification (IPC):
***D01F 8/06*** *(2006.01)* ***A61M 1/36*** *(2006.01)*
***B01D 15/38*** *(2006.01)* ***B01J 20/22*** *(2006.01)*
***D06M 13/332*** *(2006.01)*

(52) Cooperative Patent Classification (CPC):
**A61M 1/36; B01D 15/38; B01J 20/22; D01F 8/06;**
D06M 13/332

(86) International application number:
**PCT/JP2024/037294**

(87) International publication number:
**WO 2025/089209 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.10.2023 JP 2023182897**

(71) Applicant: **Toray Industries, Inc.**
**Tokyo 103-8666 (JP)**

(72) Inventors:
- **ONISHI Katsuto**
  **Otsu-shi, Shiga 520-8558 (JP)**
- **HAN Aishan**
  **Otsu-shi, Shiga 520-8558 (JP)**
- **UENO Yoshiyuki**
  **Otsu-shi, Shiga 520-8558 (JP)**
- **TAKECHI Shingo**
  **Otsu-shi, Shiga 520-8558 (JP)**
- **KANO Hidekazu**
  **Otsu-shi, Shiga 520-8558 (JP)**
- **KOGAWA Taisuke**
  **Otsu-shi, Shiga 520-8558 (JP)**
- **KADOWAKI Koji**
  **Otsu-shi, Shiga 520-8558 (JP)**

(74) Representative: **Kador & Partner Part mbB**
**Corneliusstraße 15**
**80469 München (DE)**

# (54) SEA-ISLAND COMPOSITE FIBER, CELL ADSORPTION MATERIAL, AND CELL ADSORPTION COLUMN

(57) The present invention aims at providing a sea-island composite fiber in which the generation of eluates due to decomposition of functional groups introduced into a polymer constituting a sea component is suppressed. The present invention provides a sea-island composite fiber including an island component and a sea component, wherein the sea component is fixed with a ligand and is covalently bonded with a chloro group-containing compound, and the sea-island composite fiber satisfies the following Formula 1:

$$1.3 \leq \text{spectral intensity of } {}^{35}\text{Cl}^- \text{ in region A/spectral intensity of } {}^{35}\text{Cl}^- \text{ in region B} \leq 20.0 \ldots$$

(Formula 1)

Region A: a region within 1.0 μm from outermost surface of a cross section of the sea-island composite fiber
Region B: a region within a radius of 5.0 μm from center of gravity of the cross section of the sea-island composite fiber
Spectral intensity of $^{35}Cl^-$: a spectral intensity of $^{35}Cl^-$ when a spectral intensity of $^{25}C_2H^-$ measured by time-of-flight secondary ion mass spectrometry in the respective region is defined as 1.

EP 4 800 165 A1

## Description

TECHNICAL FIELD

The present invention relates to a sea-island composite fiber with a controlled fiber structure, a cell-adsorbing material, and a cell-adsorbing column.

Background Art

Sea-island composite fibers in which a large number of island components are arranged in a sea component have been conventionally known to be used for medical applications, and examples are known such as "TORAYMYXIN (registered trademark)" (manufactured by Toray Industries, Inc.), in which a sea component is subjected to chemical treatment to impart molecular adsorption performance and used as a packing carrier for a medical extracorporeal circulation column. In addition, since fibers have a large specific surface area and can be easily processed, they are suitable as materials having molecular adsorption performance, and thus fibers targeting various adsorption targets in the medical field have been researched.

In Patent Document 1, a ligand that interacts with LAP-positive immune cells, which is a substance to be an adsorption target, is fixed on the fiber surface. By controlling the distribution of the ligand to be fixed to the sea component, LAP-positive immune cells are adsorbed with high efficiency. In addition, although a polymer of a single component alone would result in the fiber shape being destroyed and turned into a powder due to the ligand fixation reaction, an adsorbing material in which the fiber morphology is maintained is obtained by chemically treating only the sea component of the sea-island composite fiber to perform a chemical modification reaction and using a chemical-resistant polymer as the island component.

Further, in Patent Document 2, a ligand that interacts with latent TGF-β, which is a substance to be an adsorption target, is fixed on the fiber surface. By reducing the diameter of the island components and arranging a large number of them while controlling the thickness of the outermost sea component to be a certain value or less, the adhesion between the sea component and the island components is enhanced, and the generation of fine particles derived from the separation of the sea component from the island components and the destruction of the sea component deteriorated by the chemical modification reaction is suppressed.

PRIOR ART DOCUMENTS

Patent Documents

| | |
|---|---|
| Patent Document 1 | WO 2023/074729 |
| Patent Document 2 | WO 2019/045031 |

SUMMARY OF INVENTION

Problem Which the Invention Tries to Solve

However, when a polymer material is subjected to a chemical modification reaction, the polymer material may deteriorate. In particular, regarding pharmaceuticals and medical devices, since eluted foreign matter entering the body directly leads to health hazards, a reduction in the risk of generation of eluates due to material deterioration is strongly desired more than in other applications, as tests regarding eluates are specified in the Japanese Pharmacopoeia.

In the method described in Patent Document 1, since spacers for covalently bonding the polymer constituting the sea component and the ligand are distributed into the interior of the sea-island composite fiber, there is a possibility that eluates may be generated due to decomposition of functional groups introduced into the sea component by sterilization treatment or the like.

Further, in the method described in Patent Document 2, although the arrangement of the island components and the sea component is controlled to suppress the generation of fine particles derived from the separation of the sea component from the island components and the destruction of the sea component deteriorated by the chemical modification reaction, no attention is paid to the control of the structures of the polymer constituting the island components and the polymer constituting the sea component, and the control of the generation of eluates due to the decomposition of functional groups introduced into the polymer constituting the sea component has not been achieved.

Therefore, an object of the present invention is to provide a sea-island composite fiber in which the generation of eluates due to the decomposition of functional groups introduced into the polymer constituting the sea component is suppressed.

Means for Solving the Problem

The present inventors have conducted intensive studies to solve the above problems, and as a result, have found the inventions according to the following (1) to (7).

(1) A sea-island composite fiber comprising an island component and a sea component,

wherein the sea component is fixed with a ligand and is covalently bonded with a chloro group-containing compound, and
the sea-island composite fiber satisfies the following Formula 1:

$1.3 \leq$ spectral intensity of $^{35}Cl^-$ in region A/spectral intensity of $^{35}Cl^-$ in region B $\leq 20.0$ Formula 1

Region A: a region within 1.0 μm from outermost surface of a cross section of the sea-island composite fiber
Region B: a region within a radius of 5.0 μm from center of gravity of the cross section of the sea-island composite fiber Spectral intensity of $^{35}Cl^-$: a spectral intensity of $^{35}Cl^-$ when a spectral intensity of $^{25}C_2H^-$ measured by time-of-flight secondary ion mass spectrometry in the respective region is defined as 1.

(2) The sea-island composite fiber according to (1), wherein the island component consists of a crystalline polymer, and the crystalline polymer has a degree of crystallinity of 49.0% or more and a degree of orientation of 1.20 or more.
(3) The sea-island composite fiber according to (1) or (2), wherein the island component has a diameter of 0.63 μm or more and 1.25 μm or less, and distance from surface of the sea-island composite fiber to outermost island component is 1.9 μm or less.
(4) The sea-island composite fiber according to any of (1) to (3), wherein the ligand is an amino group-containing compound, and has a charge density of 0.1 μmol or more and less than 2500 μmol per 1 g of dry weight.
(5) The sea-island composite fiber according to any of (1) to (4), wherein the island component consists of a polyolefin, and the sea component contains, as a main component, a polymer selected from the group consisting of polystyrene, polysulfone, polymethyl methacrylate, and derivatives thereof.
(6) A cell-adsorbing material comprising the sea-island composite fiber according to any of (1) to (5).
(7) A cell-adsorbing column comprising the cell-adsorbing material according to (6).

Also, the present inventors have conducted intensive studies to solve the above problems, and as a result, have found the inventions according to the following (8) to (14).

(8) A sea-island composite fiber comprising an island component and a sea component, wherein the sea component is fixed with a ligand and is covalently bonded with a chloro group-containing compound, and the sea-island composite fiber satisfies the following Formula 1:

$1.3 \leq$ spectral intensity of $^{35}Cl^-$ in region A/spectral intensity of $^{35}Cl^-$ in region B $\leq 20.0$ Formula 1

Region A: a region within 1.0 μm from outermost surface of a cross section of the sea-island composite fiber Region B: a region within a radius of 5.0 μm from center of gravity of the cross section of the sea-island composite fiber Spectral intensity of $^{35}Cl^-$: a spectral intensity of $^{35}Cl^-$ when a spectral intensity of $^{25}C_2H^-$ measured by time-of-flight secondary ion mass spectrometry in the respective region is defined as 1.
(9) The sea-island composite fiber according to (8), wherein the island component consists of a crystalline polymer, and the crystalline polymer has a degree of crystallinity of 49.0% or more and a degree of orientation of 1.20 or more.
(10) The sea-island composite fiber according to (8) or (9), wherein the island component has a diameter of 0.63 μm or more and 1.25 μm or less, and distance from surface of the sea-island composite fiber to outermost island component is 1.9 μm or less.
(11) The sea-island composite fiber according to any of (8) to (10), wherein the ligand is an amino group-containing compound, and has a charge density of 0.1 μmol or more and less than 500 μmol per 1 g of dry weight.
(12) The sea-island composite fiber according to any of (8) to (11), wherein the island component consists of a polyolefin, and the sea component contains, as a main component, a polymer selected from the group consisting of

polystyrene, polysulfone, polymethyl methacrylate, and derivatives thereof.

(13) A cell-adsorbing material comprising the sea-island composite fiber according to any of (8) to (12).

(14) A cell-adsorbing column comprising the cell-adsorbing material according to (13).

EFFECTS OF INVENTION

Since the sea-island composite fiber of the present invention can suppress the generation of eluates such as chloroacetic acid, which is an acidic compound, it can be suitably used as a cell-adsorbing material for medical applications requiring high safety, and can be used as a packing material for a medical column.

Mode For Carrying Out Invention

Hereinafter, the present invention will be described in detail. The sea-island composite fiber of the present embodiment has island components and a sea component, and the sea component is fixed with a ligand and is covalently bonded to a chloro group-containing compound, and satisfies the following Formula 1.

$$1.3 \leq \text{spectral intensity of } ^{35}Cl^- \text{ in region A/spectral intensity of } ^{35}Cl^- \text{ in region B} \leq 20.0 \quad \text{Formula 1}$$

Region A: a region within 1.0 μm from the outermost surface of a cross section of the sea-island composite fiber

Region B: a region within a radius of 5.0 μm from the center of gravity of the cross section of the sea-island composite fiber

Spectral intensity of $^{35}Cl^-$: a spectral intensity of $^{35}Cl^-$ when a spectral intensity of $^{25}C_2H^-$ measured by time-of-flight secondary ion mass spectrometry in the respective region is defined as 1.

A sea-island composite fiber is one in which two or more types of polymers having different compositions form a fiber cross section in a direction perpendicular to a fiber axis. Here, the sea-island composite fiber has a cross-sectional structure in which island components composed of a polymer are scattered in a sea component composed of the other polymer. A core-sheath composite fiber is defined as a sea-island composite fiber in which the number of island components (the "number of island components" herein refers to the number of island components scattered in a fiber cross section, also referred to as "number of islands") is 1. The shape of the sea-island composite fiber is not particularly limited, but is preferably circular because it is less likely to be damaged by friction.

The fiber diameter of the sea-island composite fiber before chemical modification reaction is preferably 1 μm to 50 μm, and more preferably 2 to 30 μm. The shape of the island component is not particularly limited, and may be any shape such as a circle, an ellipse, a polygon, or a star. In the cross section of the island component in the direction perpendicular to the fiber axis of the sea-island composite fiber, the island component may be formed of two or more types of polymers having different compositions. As for the shape of the sea-island composite fiber, among fiber shapes, a fiber bundle, a yarn, a net, a knitted fabric, or a woven fabric obtained by processing the fibers is preferable, and a fiber bundle, a knitted fabric, or a woven fabric is more preferable in view of a large specific surface area and small flow resistance.

In the sea-island composite fiber of the present embodiment, the number of sea components is preferably 1. The number of island components is not particularly limited, but it is preferable that 50 or more island components are scattered in the sea component because the generation of fine particles due to mechanical damage such as friction can be suppressed by increasing the adhesion between the sea component and the island components to improve durability. On the other hand, in order to perform spinning so as to prevent joining of island components with each other, the number is preferably 1500 or less. Thus, the number of island components is preferably 50 to 1500.

The island component preferably consists of a crystalline polymer from the viewpoint of chemical resistance and mechanical strength, and for example, polyethylene terephthalate, or a copolymer obtained by copolymerizing polyethylene terephthalate with at least one selected from the group consisting of phthalic acid, isophthalic acid, 5-sodium sulfoisophthalic acid, adipic acid, sebacic acid, 1,4-butanediol, diethylene glycol, polyethylene glycol, ε-caprolactone, and lactic acid; a liquid crystal polyester such as polyarylate; a melt-moldable polymer such as polyethylene naphthalate, polyphenylene sulfide, polybutylene terephthalate, polytrimethylene terephthalate, polystyrene, polyolefin, polyacrylate, polyamide, polylactic acid, thermoplastic polyurethane, or a polymer alloy thereof is preferably used. Among these, polyolefins such as polyethylene or polypropylene are preferable from the viewpoint of chemical resistance, and polypropylene is more preferable.

The "crystalline polymer" means a polymer capable of forming a crystalline region. Here, the crystalline region refers to a portion where polymer chains are arranged and atoms are arranged three-dimensionally and periodically to form a space lattice.

The sea component preferably contains an amorphous polymer as a main component from the viewpoint of solubility in a reaction solvent, and for example, a polymer selected from the group consisting of polystyrene, polysulfone, polymethyl methacrylate, and derivatives thereof can be used, and specifically, a homopolymer of polystyrene, poly-α-methylstyrene, polychloromethylstyrene, polyethersulfone, polysulfone, polyarylethersulfone, chloromethylated polysulfone, or polymethyl methacrylate, a copolymer obtained by combining two or more of the above polymers; a copolymer of any of the monomers constituting the above polymer and a monomer other than the monomers constituting the above polymer (for example, an acrylonitrile-styrene copolymer) containing any of the above polymers as a main component; or a polymer alloy of any of the above polymers and a polymer other than the above polymer (for example, a polymer alloy of polystyrene and polypropylene) containing any of the above polymers as a main component can be used.

Especially, a polymer alloy of polystyrene and polyolefin (for example, a polymer alloy of polystyrene and polyethylene containing polystyrene as a main component, or a polymer alloy of polystyrene and polypropylene containing polystyrene as a main component) is more preferable from the viewpoint of having chemical resistance and easily maintaining a physical shape. Among these, a polymer alloy of polystyrene and polypropylene containing polystyrene as a main component is more preferable. Here, the main component means a component having the highest weight ratio among the constituent polymers.

The "amorphous polymer" means a polymer that does not form the above-described crystalline region.

The polymer constituting the island component and the polymer constituting the sea component may be combined arbitrarily. For example, the main component of the polymer constituting the sea component is a polymer selected from the group consisting of polystyrene, polysulfone, polymethyl methacrylate, and derivatives thereof, and the polymer constituting the island component is a polyolefin.

Especially, the polymer constituting the sea component is preferably a polymer alloy of polystyrene and polypropylene containing polystyrene as a main component, and the polymer constituting the island component is preferably polypropylene. Note that when the polymer constituting the island component and the polymer constituting the sea component are the same, a sea-island composite fiber cannot be formed, so the polymer constituting the island component and the polymer constituting the sea component need to have different compositions or different constituent ratios.

As for the diameter of the island component constituting the sea-island composite fiber, a smaller diameter is preferable because the surface area of the island component relative to the fiber volume increases, and the adhesion between the sea component and the island component increases, thereby suppressing the generation of eluates due to the decomposition of functional groups covalently bonded to the sea component. On the other hand, since the strength of the island component improves as the diameter of the island component increases, the diameter of the island component is 0.63 to 1.25 μm, preferably 0.70 to 1.10 μm, and more preferably 0.75 to 1.00 μm.

A smaller distance from the surface of the sea-island composite fiber to the island component is preferable because the surface layer thickness of the fragile sea component deteriorated by the chemical modification reaction in the absence of a reinforcing component becomes thinner, the adhesion between the sea component and the island component increases, and the generation of eluates due to the decomposition of functional groups covalently bonded to the sea component is suppressed.

From the above, the distance from the surface of the sea-island composite fiber to the outermost island component is 1.9 μm or less, preferably 1.5 μm or less, and more preferably 1.0 μm or less. The lower limit of the distance from the surface of the sea-island composite fiber to the outermost island component is not particularly limited and may be 0 μm. That is, the distance from the surface of the sea-island composite fiber to the outermost island component needs to be 0 μm or more and 1.9 μm or less, preferably 0 μm or more and 1.5 μm or less, and more preferably 0 μm or more and 1.0 μm or less.

In the sea-island composite fiber of the present embodiment, the sea component and a ligand are fixed in order to impart molecular adsorption performance. The "ligand" means a molecule or compound that specifically binds to a molecule present in vivo such as a protein or a cell.

In the case of fixing the sea component and the ligand, various known methods such as a physical adsorption method, an ionic bonding method, a covalent bonding method, a method of performing chemical treatment with an acid, an alkali, or the like, and a method of introducing a graft chain by heat treatment, hydrolysis, radiation, or plasma treatment can be used without any particular limitation. In the present invention, from the viewpoint of storage stability, stability, and safety of the sea-island composite fiber, it is important that the ligand does not detach, so it is preferable that the sea component and the ligand are covalently bonded to perform firm fixation.

When the sea component and the ligand are fixed by covalent bonding, the ligand needs to contain a functional group. Preferred examples of the functional group include an amino group, carboxyl group, an acid anhydride group, sulfo group, a sulfate ester group, hydroxyl group, mercapto group, silanol group, epoxy group, an ether group, phosphate group, isocyanate group, nitro group, formyl group, an amide group, imino group, succinimide group, nitrile group, pyridyl disulfide group, and a halogen group; and an amino group is more preferable from the viewpoint of ease of chemical modification reaction of the ligand and stability in blood. That is, the ligand covalently bonded to the sea component is more

preferably an amino group-containing compound.

The "amino group" as used in the present disclosure includes primary amino group, a secondary amino group, a tertiary amino group, and further a quaternary ammonium group. Further, the phrase "the sea component and the amino group-containing compound are covalently bonded" means any state as long as a component contained in the sea component and the amino group-containing compound are covalently bonded, but it is preferable that the amino group-containing compound is covalently bonded to a polymer constituting the sea component, and it is more preferable that the amino group-containing compound is indirectly covalently bonded to the polymer constituting the sea component via a chloro group-containing compound.

The amino group-containing compound is selected from monoamines and polyamines, and examples thereof include monoalkylamines such as ethylamine, propylamine, butylamine, pentylamine, hexylamine, heptylamine, octylamine, nonylamine, and decylamine; dialkylamines such as diethylamine, dipropylamine, dibutylamine, diheptylamine, dioctylamine, and dicyclohexylamine; trialkylamines such as trimethylamine, triethylamine, tripropylamine, tributylamine, tripentylamine, trihexylamine, triheptylamine, trioctylamine, trinonylamine, tridecylamine, and tridodecylamine; and polyamines such as ethylenediamine, diethylenetriamine (DETA), triethylenetetramine, tetraethylenepentamine (TEPA), dipropylenetriamine, and polyethyleneimine (PEI), among which ethylenediamine, diethylenetriamine, triethylenetetramine, tetraethylenepentamine, dipropylenetriamine, or polyethyleneimine is preferable, ethylenediamine, diethylenetriamine, triethylenetetramine, or tetraethylenepentamine is more preferable, and diethylenetriamine or tetraethylenepentamine is further preferable. Other functional groups may be substituted for the amino group possessed by the amino group-containing compound covalently bonded to the sea component.

When the above polyamine is used as the amino group-containing compound, a plurality of amino groups may be covalently bonded to the sea-island composite fiber to form a crosslinked structure. That is, when the polyamine is covalently bonded to the sea-island composite fiber as the amino group-containing compound, a crosslinked structure is formed when at least two of the amino groups in the polyamine are covalently bonded to the sea-island composite fiber.

As the chloro group-containing compound, those having an electrically neutral chemical bond such as an amide bond, urea bond, an ether bond, or an ester bond are preferable, and those having an amide bond or urea bond are more preferable. Two or more amino group-containing compounds may be covalently bonded to one chloro group-containing compound.

Examples of the chloro group-containing compound that mediates the covalent bond between the sea component and the amino group-containing compound include active chloro groups such as chloromethyl group, chloroacetyl group, chloroacetamidomethyl group, and a chloroalkyl group; epoxide group; carboxyl group; isocyanate group; thioisocyanate group; and an acid anhydride group, and from the viewpoint of having appropriate reactivity, an active chloro group (particularly chloroacetyl group) is preferable, and chloroacetamidomethyl group is more preferable. Specific examples of the polymer constituting the sea component in the sea-island composite fiber into which the chloro group-containing compound is introduced include polystyrene to which a chloroacetamidomethyl group is added, polysulfone to which a chloroacetamidomethyl group is added, and the like. These polymers are soluble in organic solvents and have an advantage of being easily molded.

The chloro group-containing compound can be introduced by previously reacting it with a polymer constituting the sea component in the sea-island composite fiber. For example, in the case where the polymer constituting the sea component is polystyrene and the reactive functional group is chloroacetamidomethyl group, polystyrene into which chloroacetamidomethyl group is introduced can be obtained by reacting polystyrene with N-methylol-a-chloroacetamide. Then, a covalent bond can be formed by reacting an amino group-containing compound (for example, diethylenetriamine or tetraethylenepentamine) with the polystyrene into which the chloroacetamidomethyl group has been introduced. In this case, the acetamidomethyl group serves as a spacer that mediates the polymer (polystyrene) constituting the sea component in the sea-island composite fiber and the amino group-containing compound.

From the viewpoint of adsorption performance of cells such as LAP-positive immune cells, it is preferable that the charge density of the amino group-containing compound covalently bonded to the sea component is higher. On the other hand, from the viewpoint of suppressing undesirable non-specific adsorption of a blood anticoagulant (heparin or the like) or the like, it is preferable that the charge density of the amino group-containing compound covalently bonded to the sea component is lower. It is known that, when used for blood purification therapy, a carrier having high heparin adsorbability has problems such as difficulty in controlling the blood heparin concentration during treatment (JP H5-329364 A).

Therefore, the charge density of the amino group-containing compound covalently bonded to the polymer constituting the sea component is 0.1 μmol or more and less than 2500 μmol per 1 g of dry weight of the sea-island composite fiber. The lower limit of the charge density of the amino group-containing compound covalently bonded to the polymer constituting the sea component is preferably 0.1 μmol or more, more preferably 1 μmol or more, and even more preferably 10 μmol or more, per 1 g of dry weight of the sea-island composite fiber. Also, the upper limit of the charge density of the amino group-containing compound covalently bonded to the polymer constituting the sea component is preferably less than 2500 μmol, more preferably 2000 μmol or less, further preferably 1500 μmol or less, even more preferably 1000 μmol or less, even more preferably less than 2500 μmol, and even more preferably 350 μmol or less, per 1

g of dry weight of the sea-island composite fiber.

When measuring the charge density, the sea-island composite fiber in a dried state is used. Here, the dried state refers to a fiber in a state in which the amount of a liquid component contained in the sea-island composite fiber is 1% by weight or less; when the fiber is dried in a vacuum dryer (0.06 atm or less) at room temperature for 12 hours after measuring the weight of the fiber and the weight loss of the remaining fiber is 1% by weight or less of the weight before drying, the fiber is considered to be in a dried state.

As a method for measuring the charge density of the sea-island composite fiber per 1 g of dry weight, for example, acid-base titration can be used.

As the degree of crystallinity of the crystalline polymer constituting the island component is higher, the density of the crystalline polymer is higher, and thus the magnitude of intermolecular interaction acting between the crystalline polymer and the amorphous polymer constituting the sea component is larger, increasing the adhesion between the island component and the sea component. This further suppresses the generation of eluates due to decomposition of functional groups covalently bonded to the sea component.

From the above, the degree of crystallinity of the crystalline polymer constituting the island component is 49.0% or more, preferably 51.0% or more, more preferably 53.0% or more, further preferably 54.5% or more, and even more preferably 56.0% or more. In addition, in order to prevent a decrease in solubility of the sea component in a reaction solvent and prevent a decrease in charge density of the amino group-containing compound covalently bonded to the amorphous polymer constituting the sea component, the degree of crystallinity of the crystalline polymer constituting the island component is preferably 100% or less.

As the degree of orientation of the crystalline polymer constituting the island component is higher, the degree of freedom of the higher-order structure of the crystalline polymer is reduced and the distance from the amorphous polymer constituting the sea component is reduced, and thus the magnitude of intermolecular interaction acting between the crystalline polymer constituting the island component and the amorphous polymer constituting the sea component is larger, increasing the adhesion between the island component and the sea component, which is preferable because the generation of eluates due to decomposition of functional groups covalently bonded to the sea component is suppressed. From the above, the degree of orientation of the crystalline polymer constituting the island component is 1.20 or more, preferably 1.30 or more, more preferably 1.40 or more, further preferably 1.55 or more, and even more preferably 1.70 or more. In addition, in order to prevent a decrease in solubility of the sea component in a reaction solvent and prevent a decrease in charge density of the amino group-containing compound covalently bonded to the amorphous polymer constituting the sea component, the degree of orientation of the crystalline polymer constituting the island component is preferably 50.0 or less.

As the elongation at maximum point of the sea-island composite fiber is larger, durability against external stress such as tension is larger, so that the generation of eluates is suppressed and process passability of chemical modification reaction and dimensional stability of a higher-order processed product are improved, which is preferable. From the above, the elongation at maximum point of the sea-island composite fiber is 3.4% or more, preferably 5.0% or more, more preferably 7.0% or more, further preferably 8.5% or more, and even more preferably 10.0% or more. In addition, if the elongation at maximum point of the sea-island composite fiber is too large, the deformability of the sea-island composite fiber increases and the dimensional stability of a higher-order processed product decreases, so it is preferably 100% or less.

As the strength at maximum point of the sea-island composite fiber is larger, durability against mechanical damage such as tension, compression, and bending is larger, so that the generation of eluates is suppressed and process passability of chemical modification reaction and dimensional stability of a higher-order processed product are improved, which is preferable. From the above, the strength at maximum point of the sea-island composite fiber is 0.13 cN/dtex or more, preferably 0.20 cN/dtex or more, more preferably 0.30 cN/dtex or more, further preferably 0.40 cN/dtex or more, and even more preferably 0.45 cN/dtex or more. In addition, if the strength at maximum point of the sea-island composite fiber is too large, durability against external force such as cutting or sectioning is too large and processability decreases, so it is preferably 10 cN/dtex or less.

In order to obtain a fiber having the degree of crystallinity, degree of orientation, elongation at maximum point, and strength at maximum point as described above, it is preferable to use a drawn yarn obtained by further drawing a yarn obtained by spinning.

The sea-island composite fiber of the present invention satisfies the following Formula 1.

$$1.3 < \text{spectral intensity of } {}^{35}\text{Cl}^- \text{ in region A/spectral intensity of } {}^{35}\text{Cl}^- \text{ in region B} < 20.0 \quad \text{Formula 1}$$

Region A: a region within 1.0 μm from outermost surface of a cross section of the sea-island composite fiber

Region B: a region within a radius of 5.0 μm from center of gravity of the cross section of the sea-island composite fiber

Spectral intensity of $^{35}Cl^-$: a spectral intensity of $^{35}Cl^-$ when a spectral intensity of $^{25}C_2H^-$ measured by time-of-flight secondary ion mass spectrometry in the respective region is defined as 1.

As the "spectral intensity of $^{35}Cl^-$ in region A / spectral intensity of $^{35}Cl^-$ in region B" increases, the generation of eluates such as chloroacetic acid, which is an acidic compound and has a risk of having a harmful effect on a living body, is suppressed, which is preferable.

Therefore, the spectral intensity of $^{35}Cl^-$ in the cross section of the sea-island composite fiber is required to satisfy the above Formula 1. The spectral intensity of $^{35}C1^-$ in region A / spectral intensity of $^{35}Cl^-$ in region B is 1.3 or more, preferably 1.5 or more, and more preferably 2.0 or more. On the other hand, it is 20.0 or less, preferably 15.0 or less, more preferably 12.0 or less, further preferably 9.0 or less, and particularly preferably 7.0 or less. Any preferable lower limit value can be combined with any preferable upper limit value.

The "spectral intensity of $^{35}Cl^-$ in region A" and the "spectral intensity of $^{35}Cl^-$in region B" can be determined by measuring a mass spectrum of $^{25}C_2H^-$ and a mass spectrum of $^{35}Cl^-$ in a cross section of the sea-island composite fiber by time-of-flight secondary ion mass spectrometry (TOF-SIMS method), mapping each intensity, and analyzing. More specifically, they can be determined by the method described in "Measurement of spectral intensity of $^{35}Cl^-$" described later.

The spectral intensity of $^{35}Cl^-$ in region A and the spectral intensity of $^{35}Cl^-$ in region B can be controlled by the amount of the chloro group-containing compound introduced into the sea component and the amount of the amino group-containing compound introduced into the sea component, the type of reaction solvent used in covalently bonding the sea component and the chloro group-containing compound, the polymer structure constituting the sea-island composite fiber, and the like.

For example, by increasing the degree of crystallinity and degree of orientation of the island component constituting the sea-island composite fiber, the spectral intensity of $^{35}Cl^-$ in region A decreases, the spectral intensity of $^{35}Cl^-$ in region B increases, and the spectral intensity of $^{35}Cl^-$ in region A / spectral intensity of $^{35}Cl^-$ in region B increases.

Since the sea-island composite fiber of the present invention can suppress the generation of eluates such as chloroacetic acid, which is an acidic compound derived from a chloro group-containing compound, it can be suitably used as an adsorbing material for medical applications where high safety is required.

The "adsorbing material" means a carrier capable of adsorbing and removing biological components (for example, proteins, cells). As the shape of the adsorbing carrier, yarn bundle, knitted fabric, or woven fabric is preferable, and knitted fabric is particularly preferable, considering that the specific surface area is large and the flow path resistance is small.

The "medical use" means a use for the purpose of treating a disease, and examples thereof include body fluid purification uses and artificial organ uses. Among them, the sea-island composite fiber of the present embodiment is preferably used for body fluid purification because of its excellent molecular adsorption capacity. Body fluid purification uses include protein adsorption uses, small molecule adsorption uses, cell adsorption uses, and the like, but the cell adsorption use is more preferable in that the molecular adsorption capacity of the sea-island composite fiber of the present embodiment can be most expected to be exhibited.

The adsorbing material of the present invention is preferably a cell-adsorbing material that adsorbs immune cells. In addition, the adsorbing material is preferably a cell-adsorbing material in which the immune cells adsorb LAP-positive immune cells. Among LAP-positive immune cells, a cell-adsorbing material that adsorbs LAP-positive platelets is preferable, and a cell-adsorbing material that adsorbs LAP-positive CD42b-positive platelets is more preferable.

In the cell-adsorbing material of the present invention, the adsorption rate of LAP-positive platelets is preferably 70% or more, more preferably 80% or more, and further preferably 90% or more. Here, the platelets are CD42b-positive platelets. Examples of a test system for the above-mentioned adsorption rate include an adsorption column flow-through type immune cell adsorption test using human blood (see the Examples). In addition, examples of an evaluation system include analysis by flow cytometry using surface antigens of immune cells as an index (see the Examples).

The cell-adsorbing column of the present invention comprises the cell-adsorbing material of the present invention.

The "cell-adsorbing column" means a column having at least a blood inlet part, a case part, and a blood outlet part, wherein the case part is filled with a cell-adsorbing material. Examples of the cell-adsorbing column include a radial flow type cell-adsorbing column.

The "radial flow type" refers to a way in which blood flows inside the column. When blood flows in a vertical direction at the inlet and outlet of the column, it is called a radial flow type when a horizontal blood flow exists inside the column.

The cell-adsorbing column of the present invention can be used for blood purification therapy. By using the cell-adsorbing column of the present invention as a blood purification column, LAP-positive immune cells and the like can be removed from blood with high efficiency while suppressing non-specific adsorption of platelets, white blood cells, and the like. For example, by circulating blood extracorporeally and passing it through the cell-adsorbing column of the present

invention, LAP-positive immune cells and the like can be removed from blood with high efficiency. That is, the cell-adsorbing column of the present invention can be used as an extracorporeal circulation column. More specifically, the cell-adsorbing column of the present invention can be used for cancer treatment in which LAP-positive immune cells are removed from the blood of cancer patients.

## EXAMPLES

Hereinafter, the present invention will be described with reference to examples, but the present invention is not limited by these examples. The following treatments and evaluations were performed for each of the examples and comparative examples.

<Preparation of Raw Knitted Fabric>

A sea-island composite fiber (fiber diameter: 20 μm) was obtained by spinning and then drawing a fiber having 264 island components made of polypropylene (manufactured by Japan Polypropylene Corporation; Novatec (registered trademark) PP FA3KM) and a sea component made of 90 wt% of polystyrene (weight average molecular weight: 181,000) and 10 wt% of polypropylene (manufactured by Japan Polypropylene Corporation; Novatec (registered trademark) PP FA3KM), wherein the ratio of islands to sea (mass ratio) was 50:50.

<Preparation of Knitted Fabric Comprising Sea-Island Composite Fiber in Which Sea Component and Amino Group-Containing Compound Are Covalently Bonded>

The obtained sea-island composite fiber was formed into a knitted fabric (hereinafter referred to as "raw knitted fabric").

After dissolving paraformaldehyde (hereinafter "PFA", 0.0704 g) in a mixed solution of 6.55 mL (12.0 g) of concentrated sulfuric acid and 10.0 mL (12.0 g) of nitrobenzene at 4°C, N-hydroxymethyl-chloroacetamide (hereinafter "NMCA", 1.69 g) was dissolved at 4°C. The raw knitted fabric (1 g) was immersed in this solution and reacted at 4°C for 2 hours. Thereafter, the reaction solution was removed, and the knitted fabric was impregnated with 17 mL (19.9 g) of ice-cooled nitrobenzene for 30 minutes for washing. Subsequently, the nitrobenzene was removed, and the knitted fabric was impregnated with 17 mL (20 g) of methanol for 30 minutes for washing to obtain a knitted fabric comprising sea-island composite fibers in which the sea component and the chloro group-containing compound were covalently bonded (hereinafter referred to as "intermediate knitted fabric").

Further, 0.747 mL (0.709 g) of diethylenetriamine was added to 13.0 mL (13.0 g) of distilled water and stirred for 30 minutes to prepare a solution. The intermediate knitted fabric was immersed in this solution and reacted at 40°C for 3 hours. Thereafter, the reaction solution was removed, and the intermediate knitted fabric was impregnated with 17 mL (17 g) of distilled water at 50°C for 30 minutes for washing, to obtain a knitted fabric comprising sea-island composite fibers in which the sea component and the amino group-containing compound were covalently bonded (hereinafter, "knitted fabric comprising sea-island composite fiber in which the amino group-containing compound is covalently bonded").

<Measurement of spectral intensity of $^{35}Cl^-$>

A knitted fabric comprising dried sea-island composite fibers was embedded in resin, and a cross section of the adsorbing material was prepared using an ultramicrotome. A cross section perpendicular to the direction of elongation of the fiber was prepared. For the entire cross section, the mass spectrum of $^{25}C_2H^-$ and the mass spectrum of $^{35}Cl^-$, which are types of negative secondary ions emitted from the cross section by primary ion irradiation, were measured using the TOF-SIMS method under the following conditions.

Measuring device: TOF.SIMS 5 (manufactured by ION-TOF)
Primary ion: Bi3++
Primary ion accelerating voltage: 30 kV
Pulse width: 125.0 ns
Secondary ion polarity: positive and negative
Number of scans: 96 scan/cycle
Cycle Time: 230 μs
Measurement range: 50 x 50 $\mu m^2$
Mass range (m/z): 0 to 800

The mass spectral intensity of $^{35}Cl^-$ when the mass spectral intensity of $^{25}C_2H^-$ in the cross section of the sea-island composite fiber was defined as 1 was mapped onto an image of the cross section of the sea-island composite fiber (Image X). At this time, in Image X, a photographing region was selected so that a cross-sectional image of one sea-island composite fiber completely fit in a field of view of 50 μm × 50 μm. Furthermore, for Image X, the following measurements were performed using image analysis software ImageJ. First, the outer periphery of the cross section of the sea-island composite fiber was surrounded and selected, and the center of gravity of the cross section of the sea-island composite fiber was measured. Next, a straight line passing through the center of gravity and vertically or horizontally crossing the cross section of the sea-island composite fiber was drawn. From two intersections of the straight line and the outer periphery of the cross section of the sea-island composite fiber, a region within 1.0 μm was defined as Region A, and a region within a radius of 5.0 μm from the center of gravity of the cross section of the sea-island composite fiber was defined as Region B. The brightness of Region A determined from Image X was defined as the spectral intensity of $^{35}Cl^-$ in Region A, and the brightness of Region B determined from Image X was defined as the spectral intensity of $^{35}Cl^-$ in Region B.

<Measurement of charge density>

The charge density of the amino group-containing compound covalently bonded to the sea component was measured by the following method. A knitted fabric composed of sea-island composite fibers was cut into a circle with a diameter of 28 mm, immersed in 40 mL of a 6 M aqueous sodium hydroxide solution, and mixed for 15 minutes. This knitted fabric was taken out, immersed in water multiple times, and mixed and washed until the pH of the liquid reached 7. The knitted fabric after washing was dried in a vacuum dryer at room temperature (0.06 atm or less) for 12 hours or more, and the weight after drying was measured (this is referred to as W [g]). This dried knitted fabric was immersed in 40 mL of 0.01 M hydrochloric acid and mixed for 30 minutes. 5 mL of this liquid was sampled, an appropriate amount of phenolphthalein solution was added, and titration was performed with a 0.005 M aqueous sodium hydroxide solution. This titration operation was performed three times, the amount of the 0.005 M aqueous sodium hydroxide solution required for each titration was measured in units of mL to the second decimal place, and the average value was defined as A [mL].

The charge density of the amino group-containing compound was calculated by the following Formula 2, and a value rounded to the nearest integer was used. Hereinafter, the charge density of the amino group-containing compound covalently bonded to the sea component per 1 g of dry weight of the sea-island composite fiber is expressed in units of μmol/g.

$$\text{Charge density } [\mu\text{mol/g}] = \{(5\ [\text{mL}] \times 0.01\ [\text{M}] - A\ [\text{mL}] \times 0.005\ [\text{M}]) \times (40\ [\text{mL}] / 5\ [\text{mL}]) \times 10^3\} / W\ [\text{g}] \qquad \text{Formula 2}$$

<Measurement of diameter of island component>

As a method for measuring the diameter of the island component of the sea-island composite fiber, a sample obtained by cutting the sea-island composite fiber perpendicular to the longitudinal direction was photographed at 10 locations for each fiber with a scanning electron microscope (SEM) S-5500 (manufactured by Hitachi High-Technologies) at a magnification at which the island component can be clearly observed. From the obtained image, the diameter of each island component was measured in units of μm to the third decimal place using image analysis software ImageJ, and the value obtained by rounding the average value of 10 locations to the second decimal place was defined as the island component diameter.

<Measurement of distance from surface of sea-island composite fiber to outermost island component>

As a method for measuring the distance from the surface of the sea-island composite fiber to the outermost island component, the image photographed as described above was divided into six sections so as to have a radial and equal cross-sectional area from the center of gravity of the fiber cross section, and the shortest distance from the fiber surface to the outermost island component in each divided fiber cross section was measured in units of μm to the first decimal place.

However, when the island component protruded from the fiber surface, it was set to 0 μm. In addition, when the island component spans multiple divided fiber cross sections and the island component is the outermost island component in multiple divided fiber cross sections, the shortest distance from the fiber surface to the island component in each divided fiber cross section was measured. The average value of the distances measured at 10 locations by the above method in each divided fiber cross section was defined as the distance from the surface of the sea-island composite fiber to the outermost island component.

<Measurement of pH and amount of eluate>

A knitted fabric composed of dried sea-island composite fibers was cut into a circle with a diameter of 28 mm,

placed in a clean container, and subjected to steam sterilization treatment by the following method. 40 mL of distilled water was added to the knitted fabric cut into a circle, and heat treatment was performed in saturated steam at 121°C for 30 minutes using an autoclave device (TOMY LDX500; manufactured by Tomy Seiko Co., Ltd.).

The pH of the obtained solution (hereinafter "solution after steam sterilization") and the amount of chloroacetic acid in the solution after steam sterilization (hereinafter "amount of eluate") were measured.

The pH was measured under the following conditions. The electrode of a pH meter (LAQUA, HORIBA) calibrated with standard solutions of pH 4 and pH 7 was immersed in 10 mL of the solution after steam sterilization, and the value when the displayed value became stable for 1 minute was taken as the pH.

The pH of the solution after steam sterilization was evaluated in two stages according to the following criteria. If the pH is 5.00 or more, it is considered that the amount of eluate is almost the same as that of the raw knitted fabric.

◦: pH 5.00 or more
△: pH 4.80 or more and less than 5.00
×: pH less than 4.80

The amount of eluate was measured under the following conditions. 10 mL of the solution after steam sterilization was treated with a solid-phase extraction cartridge, and the chloroacetic acid component concentration (μg/mL) per 1 mL of the solution was quantitatively analyzed using an ion chromatography method under the following conditions.

Measuring device: ICS-6000 (manufactured by Thermo Fisher Scientific)
Solid-phase extraction cartridge: InertSep Slim-J PLS-3 (manufactured by GL Sciences)
Separation column: IonPac AS11-HC-4 μm (2 mmφ × 250 mm)
Eluent: potassium hydroxide / gradient
Sample injection volume: 100 μL
Detector: electrical conductivity detector

The amount of eluate of the solution after steam sterilization was evaluated in two stages according to the following criteria. If the amount of eluate is less than 1.0 μg/mL, it is considered that the amount of eluate is almost the same as that of the raw knitted fabric. ◦: less than 1.0 μg/mL

△: 1.0 μg/mL or more and less than 1.5 μg/mL
×: 1.5 μg/mL or more

<Adsorption test of LAP-positive platelets>

The adsorption rate of LAP-positive immune cells in the knitted fabric composed of sea-island composite fibers was measured by an adsorption column flow-through type immune cell adsorption test using human blood. For the analysis, flow cytometry (FACSLyric, manufactured by Becton Dickinson) was used.

First, a filling part (diameter 10 mm, height 14 mm) of a cell-adsorbing column was filled with a knitted fabric composed of sea-island composite fibers with a dry mass of 0.3 g and physiological saline, and steam sterilization treatment (121°C, 30 minutes) was performed.

Thereafter, heparin-added physiological saline having a final concentration of 50 units/mL was passed through the cell-adsorbing column at 1.54 mL/min for 2.5 minutes for priming treatment. Next, blood collected from a healthy human (heparin was added to a final concentration of 5 units/mL) was passed through at 0.31 mL/min, and blood at the column outlet was collected after 30 minutes. Blood on the column inlet side was also collected after 30 minutes.

100 μL of the collected blood was dispensed into a 5-mL polypropylene tube (manufactured by FALCON), and various fluorescently labeled antibodies were added to stain the immune cells. As the various fluorescently labeled antibodies, a phycoerythrin (hereinafter referred to as "PE")-labeled anti-human CD42b antibody (manufactured by BioLegend) and an allophycocyanin (hereinafter referred to as "APC")-labeled anti-human LAP antibody (manufactured by R&D Systems) were used.

A flow cytometer was used to detect the number of LAP-positive immune cells. CD42b-positive and LAP-positive cells were evaluated as LAP-positive platelets, and the adsorption rate of LAP-positive platelets was calculated by the following Formula 3. Adsorption rate of LAP-positive platelets (%) = (1 - number of LAP-positive platelets in blood to which cell-adsorbing material was added / number of LAP-positive platelets in blood to which cell-adsorbing material was not added) × 100 ... Formula 3

The adsorption rate of LAP-positive platelets was evaluated in two stages according to the following criteria. If the adsorption rate of LAP-positive platelets is 90% or more, it is considered to sufficiently show effectiveness as a cell-

adsorbing material.

◦ 90% or more
×: less than 90%

<Evaluation of degree of crystallinity and degree of orientation of island component>

The degree of crystallinity and degree of orientation of the crystalline polymer constituting the island component were evaluated by the following method. For the dried knitted fabric composed of sea-island composite fibers, Raman spectra were measured using laser Raman spectroscopy under the following conditions.
Measuring device: near-infrared Raman spectrometer (manufactured by Photon Design)
Measurement mode: microscopic Raman
Objective lens: ×100
Beam diameter: 1 μm
Cross slit: 1000 μm
Light source: YAG laser / 1064 nm
Laser power: 2000 mW
Diffraction grating: Single 300 gr/mm
Slit: 100 μm
Detector: InGaAs / manufactured by Roper Scientific Japan

The measurement for evaluation of the degree of crystallinity of the crystalline polymer constituting the island component was performed under unpolarized conditions. In the Raman spectrum obtained by the above method, the band width of the Raman band at 400 $cm^{-1}$ was analyzed, and the degree of crystallinity of the crystalline polymer constituting the island component was calculated using the following Formula 4. As the degree of crystallinity of the crystalline polymer constituting the island component, a value rounded to the first decimal place was used.

Degree of crystallinity of crystalline polymer constituting island component (%) = {-0.0681 × (band width of Raman band at 400 $cm^{-1}$ [$cm^{-1}$]) + 1.0591} × 100 Formula 4

The measurement for evaluation of the degree of orientation of the crystalline polymer constituting the island component was performed under polarized conditions. In the Raman spectrum obtained by the above method, the ratio of the Raman band intensity at 810 $cm^{-1}$ ($I_{810}$) to the Raman band intensity at 840 $cm^{-1}$ ($I_{840}$) was used, and the ratio of the intensity ratio in parallel polarization with respect to the fiber axis ($(I_{810}/I_{840})_0$) to the intensity ratio in perpendicular polarization ($(I_{810}/I_{840})_{90}$) was taken as the degree of orientation of the crystalline polymer constituting the island component in the fiber axis direction. That is, the degree of orientation of the crystalline polymer constituting the island component was calculated by the following Formula 5. As the degree of orientation of the crystalline polymer constituting the island component, a value rounded to the second decimal place was used.

Degree of orientation of crystalline polymer constituting island component = $(I_{810}/I_{840})_0$ / $(I_{810}/I_{840})_{90}$ ... Formula 5

<Measurement of elongation at maximum point and strength at maximum point of sea-island composite fiber>

In accordance with JIS L1013 (2010), measurement was performed five times using Tensilon UCT-100 manufactured by Orientec, and the values obtained by arithmetically averaging the elongation and strength at the maximum point were used.

Example 1

After a fiber (hereinafter referred to as "Fiber of Example 1 ") was obtained by the method described above, spinning was performed, and a raw knitted fabric was prepared from sea-island composite fibers obtained by setting the draw ratio to 3.1 times. In addition, a knitted fabric composed of sea-island composite fibers in which an amino group-containing compound was covalently bonded (hereinafter referred to as "Knitted fabric of Example 1 ") was prepared by the method described above. The band width of the Raman band at 400 $cm^{-1}$ was 7.20 $cm^{-1}$, $I_{810}$ and $I_{840}$ in parallel polarization were 1817 and 1037, respectively, and $I_{810}$ and $I_{840}$ in perpendicular polarization were 1315 and 1298,

respectively.

Example 2

Using the Fiber of Example 1, a raw knitted fabric was prepared from sea-island composite fibers obtained by changing the draw ratio after spinning to 3.3 times drawing. Further, the sea component and the amino group-containing compound were covalently bonded to the raw knitted fabric in the same manner as in Example 1 to prepare a knitted fabric composed of sea-island composite fibers in which the amino group-containing compound was covalently bonded (hereinafter referred to as "Knitted fabric of Example 2"). The band width of the Raman band at 400 $cm^{-1}$ was 6.58 $cm^{-1}$, $I_{810}$ and $I_{840}$ in parallel polarization were 2052 and 990, respectively, and $I_{810}$ and $I_{840}$ in perpendicular polarization were 1279 and 1555, respectively.

Comparative Example 1

Using the Fiber of Example 1, a raw knitted fabric was prepared from sea-island composite fibers obtained without drawing. Further, the sea component and the amino group-containing compound were covalently bonded to the raw knitted fabric in the same manner as in Example 1 to prepare a knitted fabric composed of sea-island composite fibers (hereinafter referred to as "Knitted fabric of Comparative Example 1", the same as the knitted fabric composed of sea-island composite fibers in which an amino group-containing compound is covalently bonded described in the Examples of Patent Document 1). The band width of the Raman band at 400 $cm^{-1}$ was 8.46 $cm^{-1}$, $I_{810}$ and $I_{840}$ in parallel polarization were 1591 and 1274, respectively, and $I_{810}$ and $I_{840}$ in perpendicular polarization were 1217 and 1118, respectively.

Example 3

From the Fiber of Example 1, a fiber was prepared by changing it to a fiber having 264 island components made of polypropylene (manufactured by Japan Polypropylene Corporation; Novatec (registered trademark) PP FA3KM) and a sea component made of 100 wt% of polystyrene (weight average molecular weight: 181,000), with a ratio of island to sea (mass ratio) of 50:50. Thereafter, a raw knitted fabric was prepared from sea-island composite fibers (fiber diameter: 20 μm) obtained by changing the draw ratio after spinning to 3.1 times from the method of Example 1. Further, the sea component and the amino group-containing compound were covalently bonded to the raw knitted fabric in the same manner as in Example 1 to prepare a knitted fabric composed of sea-island composite fibers in which the amino group-containing compound was covalently bonded (hereinafter referred to as "Knitted fabric of Example 3"). The band width of the Raman band at 400 $cm^{-1}$ was 7.30 $cm^{-1}$, $I_{810}$ and $I_{840}$ in parallel polarization were 1802 and 1010, respectively, and $I_{810}$ and $I_{840}$ in perpendicular polarization were 1302 and 1248, respectively.

Example 4

From the Fiber of Example 1, a fiber was prepared by changing it to a fiber having 264 island components made of polypropylene (manufactured by Japan Polypropylene Corporation; Novatec (registered trademark) PP FA3KM) and a sea component made of 85 wt% of polystyrene (weight average molecular weight: 181,000) and 15 wt% of polypropylene (manufactured by Japan Polypropylene Corporation; Novatec (registered trademark) PP FA3KM), with a ratio of island to sea (mass ratio) of 50:50. Thereafter, a raw knitted fabric was prepared from sea-island composite fibers (fiber diameter: 20 μm) obtained by changing the draw ratio after spinning to 3.1 times from the method of Example 1. Further, the sea component and the amino group-containing compound were covalently bonded to the raw knitted fabric in the same manner as in Example 1 to prepare a knitted fabric composed of sea-island composite fibers in which the amino group-containing compound was covalently bonded (hereinafter referred to as "knitted fabric of Example 4"). The band width of the Raman band at 400 $cm^{-1}$ was 7.04 $cm^{-1}$, $I_{810}$ and $I_{840}$ in parallel polarization were 1882 and 1045, respectively, and $I_{810}$ and $I_{840}$ in perpendicular polarization were 1293 and 1242, respectively.

Example 5

A knitted fabric composed of sea-island composite fibers in which the amino group-containing compound was covalently bonded (hereinafter referred to as "Knitted fabric of Example 5") was prepared in the same manner as in Example 1 except that the amount of diethylenetriamine used was changed to 4.482 mL (4.254 g) in the method for covalently bonding the sea component and the amino group-containing compound for the Knitted fabric of Example 1. The band width of the Raman band at 400 $cm^{-1}$ was 7.17 $cm^{-1}$, $I_{810}$ and $I_{840}$ in parallel polarization were 1820 and 1033, respectively, and $I_{810}$ and $I_{840}$ in perpendicular polarization were 1314 and 1313, respectively.

For the Knitted fabrics of Examples 1 to 5 and Comparative Example 1, each measurement was performed by the

method described above. Table 1 shows the measurement results of the elongation at maximum point and the strength at maximum point, Table 2 shows the measurement results of the spectral intensity of $^{35}Cl^-$, the degree of crystallinity of the island component, the degree of orientation of the island component, the charge density, the diameter of the island component, and the distance from the fiber surface to the outermost island component, and Table 3 shows the measurement and evaluation results of the pH of the solution after steam sterilization, the amount of eluate, and the adsorption rate of LAP-positive platelets.

[Table 1]

| | Elongation at Maximum Point (%) | Strength at Maximum Point (cN/dtex) |
|---|---|---|
| Example 1 | 12.3 | 0.65 |
| Example 2 | 10.9 | 0.47 |
| Comparative Example 1 | 3.3 | 0.12 |
| Example 3 | 8.6 | 0.67 |
| Example 4 | 14.1 | 0.71 |
| Example 5 | 12.6 | 0.66 |

[Table 2]

| | Spectral intensity of $^{35}Cl^-$ in region A/spectral intensity of $^{35}Cl^-$ in region B | Degree of crystallinityof island component (%) | Degree of orientation of island component | Charge density (μmol/g) | Diameter of island component(μm) | Distance from surface to outermost island component (μm) |
|---|---|---|---|---|---|---|
| Example 1 | 3.81 | 56.9 | 1.73 | 312 | 0.87 | 0.9 |
| Example 2 | 2.31 | 61.1 | 2.52 | 263 | 0.86 | 1.0 |
| Comp. Example 1 | 1.21 | 48.3 | 1.15 | 167 | 0.86 | 0.9 |
| Example 3 | 6.82 | 56.2 | 1.71 | 342 | 0.86 | 0.9 |
| Example 4 | 19.23 | 58.0 | 1.73 | 220 | 0.87 | 0.9 |
| Example 5 | 3.53 | 57.1 | 1.76 | 958 | 0.87 | 1.0 |

[Table 3]

| | pH | | Amount of Eluate | | Adsorption Rate of LAP-positive platelets | | |
|---|---|---|---|---|---|---|---|
| | Value | Evaluation | (μg/mL) | Evaluation | (%) | Evaluation | |
| Example 1 | 5.58 | ○ | 0.73 | ○ | 91 | ○ | |
| Example 2 | 5.56 | ○ | 0.75 | ○ | 91 | ○ | |
| Comp. Example 1 | 3.81 | × | 2.92 | × | 90 | ○ | |
| Example 3 | 5.52 | ○ | 0.76 | ○ | 93 | ○ | |
| Example 4 | 4.92 | Δ | 1.32 | Δ | 90 | ○ | |
| Example 5 | 5.56 | ○ | 0.75 | ○ | 92 | ○ | |

[INDUSTRIAL APPLICABILITY]

The sea-island composite fiber of the present invention can be used as a cell-adsorbing material because it can adsorb target cells while suppressing the generation of eluates.

## Claims

1. A sea-island composite fiber comprising an island component and a sea component,

   wherein the sea component is fixed with a ligand and is covalently bonded with a chloro group-containing compound, and
   the sea-island composite fiber satisfies the following Formula 1:

   $$1.3 \leq \text{spectral intensity of } {}^{35}\text{Cl}^{-} \text{ in region A/spectral intensity of } {}^{35}\text{Cl}^{-} \text{ in region B} \leq 20.0 \ldots$$

   Formula 1

   Region A: a region within 1.0 μm from outermost surface of a cross section of the sea-island composite fiber
   Region B: a region within a radius of 5.0 μm from center of gravity of the cross section of the sea-island composite fiber
   Spectral intensity of $^{35}Cl^-$: a spectral intensity of $^{35}Cl^-$ when a spectral intensity of $^{25}C_2H^-$ measured by time-of-flight secondary ion mass spectrometry in the respective region is defined as 1.

2. The sea-island composite fiber according to claim 1,

   wherein the island component consists of a crystalline polymer, and
   the crystalline polymer has a degree of crystallinity of 49.0% or more and a degree of orientation of 1.20 or more.

3. The sea-island composite fiber according to claim 1 or 2, wherein the island component has a diameter of 0.63 μm or more and 1.25 μm or less, and distance from surface of the sea-island composite fiber to outermost island component is 1.9 μm or less.

4. The sea-island composite fiber according to any one of claims 1 to 3, wherein the ligand is an amino group-containing compound, and has a charge density of 0.1 μmol or more and less than 2500 μmol per 1 g of dry weight.

5. The sea-island composite fiber according to any one of claims 1 to 4,

   wherein the island component consists of a polyolefin, and
   the sea component contains, as a main component, a polymer selected from the group consisting of polystyrene, polysulfone, polymethyl methacrylate, and derivatives thereof.

6. A cell-adsorbing material comprising the sea-island composite fiber according to any one of claims 1 to 5.

7. A cell-adsorbing column comprising the cell-adsorbing material according to claim 6.

## INTERNATIONAL SEARCH REPORT

International application No.
**PCT/JP2024/037294**

**A. CLASSIFICATION OF SUBJECT MATTER**

***D01F 8/06***(2006.01)i; ***A61M 1/36***(2006.01)i; ***B01D 15/38***(2006.01)i; ***B01J 20/22***(2006.01)i; *D06M 13/332*(2006.01)n
FI: D01F8/06; A61M1/36 165; B01D15/38; B01J20/22 B; D06M13/332

According to International Patent Classification (IPC) or to both national classification and IPC

**B. FIELDS SEARCHED**

Minimum documentation searched (classification system followed by classification symbols)

D01F8/06; A61M1/36; B01D15/38; B01J20/22; D06M13/332

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2003-225304 A (TORAY INDUSTRIES, INC.) 12 August 2003 (2003-08-12) paragraphs [0059]-[0064], examples | 1-2, 4-7<br>3 |
| Y | WO 2019/045031 A1 (TORAY INDUSTRIES, INC.) 07 March 2019 (2019-03-07) paragraphs [0024]-[0025] | 3 |
| A | WO 2023/074729 A1 (TORAY INDUSTRIES, INC.) 04 May 2023 (2023-05-04) entire text | 1-7 |
| A | JP 2008-6171 A (KANSAI TECHNOLOGY LICENSING ORGANIZATION CO., LTD.) 17 January 2008 (2008-01-17) entire text | 1-7 |
| A | JP 2004-194720 A (TORAY INDUSTRIES, INC.) 15 July 2004 (2004-07-15) entire text | 1-7 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2024** | **07 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/037294**

| Patent document cited in search report | Publication date (day/month/year) | Patent family member(s) | Publication date (day/month/year) |
|---|---|---|---|
| JP 2003-225304 A | 12 August 2003 | (Family: none) | |
| WO 2019/045031 A1 | 07 March 2019 | US 2021/0154642 A1<br>paragraphs [0028]-[0029]<br>EP 3677710 A1<br>CN 110945166 A<br>KR 10-2020-0042441 A | |
| WO 2023/074729 A1 | 04 May 2023 | EP 4427768 A1<br>entire text<br>CN 118201657 A | |
| JP 2008-6171 A | 17 January 2008 | (Family: none) | |
| JP 2004-194720 A | 15 July 2004 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2023074729 A
- WO 2019045031 A
- JP H5329364 A